(19) Europäisches Patentamt
European Patent Office
Office européen des brevets

(11) Veröffentlichungsnummer: **0 383 170**
**A2**

(12) # EUROPÄISCHE PATENTANMELDUNG

(21) Anmeldenummer: **90102364.8**

(22) Anmeldetag: **07.02.90**

(51) Int. Cl.[5]: **A61K 47/48, A61K 31/505, A61K 9/18**

Patentansprüche für folgende Vertragsstaaten: ES + GR.

(30) Priorität: **11.02.89 DE 3904119**

(43) Veröffentlichungstag der Anmeldung: **22.08.90 Patentblatt 90/34**

(84) Benannte Vertragsstaaten: **AT BE CH DE ES FR GB GR IT LI NL SE**

(71) Anmelder: **HOECHST AKTIENGESELLSCHAFT**
**Postfach 80 03 20**
**D-6230 Frankfurt am Main 80(DE)**

(72) Erfinder: **Bader, Hubert, Dr.**
**4039 Carmel Forest Drive**
**Charlotte, NC 28226(US)**
Erfinder: **Magerstädt, Michael, Dr.**
**Rheingaustrasse 19**
**D-6238 Hofheim am Taunus(DE)**
Erfinder: **Schlingmann, Merten, Dr., Prof.**
**Schneidhainer Strasse 32a**
**D-6240 Königstein/Taunus(DE)**
Erfinder: **Walch, Axel, Dr.**
**Hans-Sachs-Strasse 5**
**D-6000 Frankfurt am Main 90(DE)**
Erfinder: **Gronau, Thomas, Dr.**
**Im Stetefeld 8**
**D-3551 Lahntal-Caldern(DE)**
Erfinder: **Hoffmann, Dieter, Dr.**
**Im Stetefeld 6**
**D-3551 Lahntal-Caldern(DE)**
Erfinder: **Kraemer, Hans-Peter, Dr.**
**Birkenweg 16**
**D-3550 Marburg(DE)**
Erfinder: **Sedlacek, Hans-Harald, Dr.**
**Sonnenhang 3**
**D-3550 Marburg(DE)**

(54) **Polymerfixiertes Methotrexat, Verfahren zur Herstellung und Verwendung.**

(57) Durch die Vernetzung der Carboxylgruppen von Methotrexat oder Analoga mit Hydroxylgruppen von wasserlöslichen, biokompatiblen Polymeren entsteht eine "Pro-Drug" Form des Wirkstoffs mit guten relevanten Eigenschaften für die Anti-Tumor-Humantherapie.

EP 0 383 170 A2

## Polymerfixiertes Methotrexat, Verfahren zur Herstellung und Verwendung

Weltweit werden seit Jahren Forschungsarbeiten zur Entwicklung von pharmakologisch aktiven Polymeren getätigt, insbesondere auch von polymeren Anti-Tumor-Agentien (US 4460560, US 4551502, EP 0040506, WO 875031, DE 3515178, BE 902344 und EP 0111388).

Ein wesentliches Ziel ist dabei die Herstellung von Cytostatika mit verringerten oder eliminierten Nebenwirkungen und verbesserter therapeutischer Breite. Die Probleme der derzeit klinisch angewandten Cytostatika liegen weniger in nicht ausreichender Cytotoxizität, sondern vielmehr in zu geringer Selektivität begründet. Dies bedeutet, daß die große Palette der stark cytotoxischen Verbindungen nicht nur in Tumorzellen, sondern auch in gesunden Körperzellen und in vielen Fällen auch noch verstärkt in einzelnen Organen wirken.

Wünschenswert ist daher eine Darreichungsform, in der man über längere Zeit ein Blutniveau an Cytostatikum erreichen und erhalten kann, das im Bereich der therapeutischen Breite liegt, d.h. mit dem Tumorzellen verstärkt, gesunde Körperzellen aber nur gering oder gar nicht geschädigt werden, um eine erhöhte Tumortoxizität bei gleichzeitiger verringerter Gesamtoxizität mit insgesamt wesentlich niedrigeren Dosen an Chemotherapeutikum zu erreichen.

Bisher wurden unterschiedliche Entwicklungen verfolgt:

-Polymere als Cytostatika (Polyanionen, wie Pyran-Copolymere, Polyvinylsulfonate etc.), die per se tumorhemmende Wirkung aufweisen sollen. Häufiger Nachteil: toxische Nebenwirkungen und enge Begrenzung im Molekulargewichtsbereich (Nephrotoxizität > MW 50 KD).

-Polymere mit cytostatischer Wirkung, in denen Bestandteile des "Polymer-Rückgrads" nach Abbau gewisse Wirksamkeit zeigen. Häufige Nachteile: begrenzte Auswahl der Polymerbausteine, Immunogenität, Nebenwirkungen der Polymere und vor allem die Unlöslichkeit der Produkte in Wasser (J 58174409, DE 3026448, DE 3515178, DE 3026574, BE 902344 und DE 3026575).

-Polymerfixierte Cytostatika an funktionellen Amid-Seitengruppen (WO 875031, EP 0111388, DE 3539951, WO 8700056, EP 0190464, US 4551502, JP 57143325, EP 0040506 und JP 57143326).

Die Freisetzung dieser Cytostatika aus der Bindung mit dem Polymer kann zwar theoretisch bei Endocytose des gesamten Konjugats im sauren pH des Lysosoms erfolgen, doch findet eine Endocytose, wie die Praxis zeigt, selbst bei zielspezifisch modifizierten Polymerkonjugationen (z.B. Antikörperkonjugaten) nur in geringem Maße statt. Desweiteren werfen die Einführung von Spacern, die oft geringe Beladungsdichte und schwierige Synthesen große Probleme auf. Die in vivo Ergebnisse mit solchen Konjugaten waren daher bisher enttäuschend.

Einen besonderen Fall stellen phasenspezifische Cytostatika, z.B. Methotrexat dar, da sie nur in einer bestimmten Phase des Zellzyklus (Synthesephase beim Dihydrofolatreduktasehemmer Methotrexat) wirken. Eine einmalige Applikation hat bei der relativ kurzen biologischen Halbwertzeit des Methotrexats oft überhaupt keine Anti-Tumor-Wirkung. Erst mehrfache Einzelapplikationen mit sehr hohen Dosen zeigen einen therapeutischen Effekt. Oft ist eine "Rettungstherapie" mit Antagonisten nötig, um dann zeitverzögert in den gesunden Zellen zu große Schäden zu vermeiden.

Wegen dieser Problematik ist eine polymerfixierte Form von Methotrexat von besonderem Interesse. Jedoch war durch Fixieren von Methotrexat an Poly-L-lysin, Poly-lactid-glycolid (PLG)-Derivate etc. (siehe WO 875031) bisher keinerlei befriedigende Lösung möglich.

Überraschend wurden nun durch Fixierung von Methotrexat an wasserlösliche, hydroxylgruppentragende biokompatible Polymere sehr gute in vitro und in vivo Eigenschaften mit Relevanz für die Humantherapie erreicht.

Die Erfindung betrifft somit:

1. Ein polymerfixiertes Methotrexat oder Methotrexatderivate, gekennzeichnet durch

- eine Esterbindung der $\alpha$- und/oder $\gamma$-Carboxylgruppe des Methotrexat oder der Methotrexatderivate mit Hydroxylgruppen von wasserlöslichen biokompatiblen Polymeren;
- Bewirkung einer Lebensverlängerung von mehr als 125 % gegenüber unbehandelten Kontrollen in Mäusen mit L1210-Leukämie bei einmaliger intraperitonealer Applikation von 60 mg Methotrexat oder Methotrexatderivat, das an oben genanntes Polymer fixiert ist, pro kg Körpergewicht.

2. Ein Verfahren zur Herstellung des unter 1. charakterisierten polymerfixierten Methotrexat oder der polymerfixierten Methotrexatderivate, das dadurch gekennzeichnet ist, daß das $\alpha$- und/oder $\gamma$-Carboxylgruppen tragende Methotrexat oder die entsprechenden Derivate des Methotrexats in Gegenwart wasserentziehender Kopplungsreagenzien mit einem hydroxylgruppentragenden wasserlöslichen biokompatiblen Polymeren zur Reaktion gebracht werden.

3. Die Verwendung des unter 1. charakterisierten polymerfixierten Methotrexat oder der polymerfixierten Methotrexatderivate als Tumortherapeutikum.

Im folgenden wird die Erfindung, insbesondere in ihren bevorzugten Ausführungsformen, ausführlich beschrieben. Ferner wird die Erfindung durch den Inhalt der Ansprüche definiert.

Methotrexat oder N-[4-[[(2,4-diamino-6-pteridinyl)-methyl]methylamino]benzoyl]-glutaminsäure ist als Verbindung mit ihren wesentlichen Charakteristika in dem Artikel von A.R. Chamberlain et al. in Analytical Profiles of Drug Substances, Band 5, Herausgeber K. Florey, Academic Press, New York (1976), Seiten 283-306, dargestellt. Die Herstellung der Verbindung wird von Seeger et al. [J. Am. Soc. 71, 1753 (1949) oder Rahmann et al., Medic. Res. Rev., 8, 95 (1988)] beschrieben.

Als Methotrexat Derivate oder Analoga können Verbindungen eingesetzt werden, die am Pteridinring oder in der Brücken-Region modifiziert wurden, aber auch Verbindungen, die am aromatischen Ring oder im Glutaminsäureanteil derivatisiert wurden. Wesentliche Bedingung ist jedoch, daß mindestens eine zur Bindung an das Polymer notwendige Carboxylgruppe erhalten bleibt. Derartige Analoga und deren Herstellungsverfahren sind in dem Artikel von Rahmann et al. (s.o.) ausführlich beschrieben. Besonders bevorzugt können als Derivate Methopterin (Merck Index 10 (1983) 5860) und Aminopterin (Merck Index 10 (1983) 477) verwendet werden.

Die genannten Verbindungen können an wasserlösliche, biokompatible, hydroxylgruppentragende Polymere über eine Esterbindung fixiert werden. Unter biokompatiblen Polymeren versteht man Verbindungen, die physiologisch verträglich sind sowie im Organismus abbaubar sind und/oder ausgeschieden werden können. Es können vorzugsweise Polymere verwendet werden, in denen der Anteil ionisierbarer Gruppen vor der Beladung mit dem Wirkstoff unter 10 Mol-% liegt.

Bevorzugt werden wasserlösliche Stärken mit einem mittleren Molekulargewicht von 1000 bis 200.000, vorzugsweise 5000 bis 50.000, eingesetzt, die auch modifiziert sein können. Weiterhin werden Celluloseacetate, insbesondere wasserlösliches Celluloseacetat der Firma (Celanese/WSCA), oder Dextrane, mit einem mittleren Molekulargewicht zwischen 1000 und 200.000, insbesondere mit einem mittleren Molekulargewicht von 40.000 bis 70.000, oder Inulin verwendet.

Auch entsprechende synthetische Polymere können Verwendung finden. Ein Beispiel ist das Poly-α,β-(hydroxyethyl)-D,L-aspartamid der Formel I,

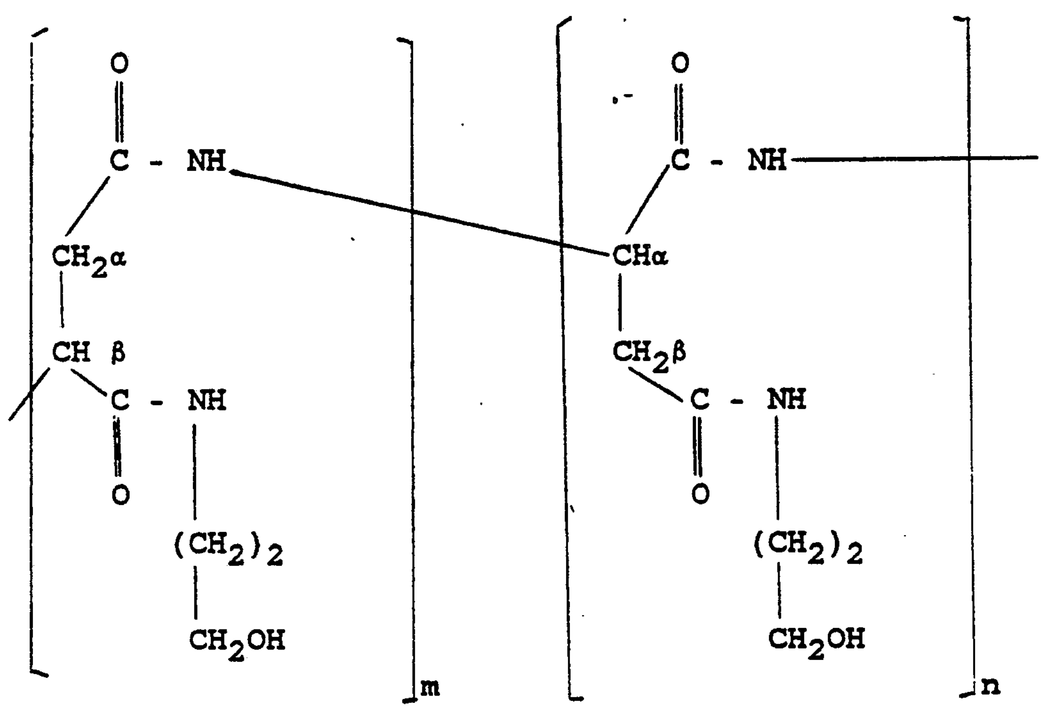

I

in der das Verhältnis von m zu n im Bereich 0:1 bis 1:0, bevorzugt 0,7: 0,3 bis 0,95 : 0,05 liegt. Das Molekulargewicht derartiger Verbindungen liegt ca. zwischen 2000 und 100.000, bevorzugt zwischen 5000 und 50.000. Entsprechende Verbindungen und deren Herstellungsverfahren werden in der Deutschen Offenlegungsschrift 37 00 128 ausführlich beschrieben.

Ein anderes Beispiel für ein synthetisches Polymer ist amidiertes Polylysinfumaramid/Polylysinglutaramid der Formel II,

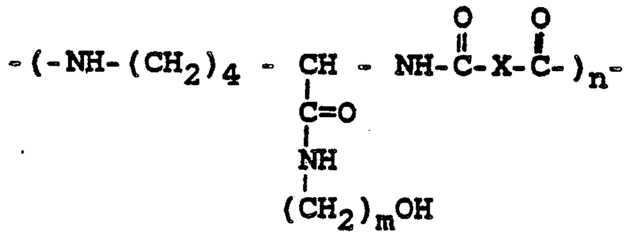

II

in der X -$(CH_2)_3$ oder -CH = CH-, m die Zahlen 1 bis 10, bevorzugt 2, und n die Zahl 5 bis 2000, bedeuten kann. Diese Polymere haben ein Molekulargewicht zwischen 1000 und 300.000. Derartige Polymere und deren Herstellungsverfahren werden in der Deutschen Offenlegungsschrift 37 07 369 beschrieben.

Ein weiteres Beispiel ist ein Copolymer der Formel III aus Poly-α,β-(2-hydroxyethyl)-D,L-aspartamid (Verbindung der Formel I) und Polysuccinimid,

(Formel I)$_y$ 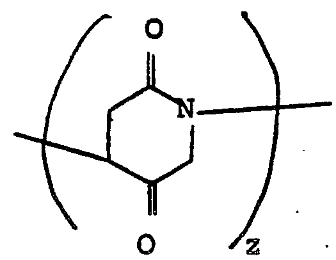

III

in der das Verhältnis von y zu z im Bereich von etwa 0,99:0,01 bis 0,01:0,99, bevorzugt 2,5:7,5 bis 7,5:2,5, liegt.

Insbesondere bevorzugt werden als Polymere nach dem erfindungsgemäßen Verfahren die in den Formeln I, II und III dargestellten synthetischen Verbindungen eingesetzt.

Zur Herstellung des polymerfixierten Methotrextrats bzw. der entsprechenden Derivate wird der Wirkstoff gelöst, beispielsweise in Wasser, Dimethylsulfoxid (DMSO), Formamid, N,N-Dimethylformamid oder Methylenchlorid oder einem Gemisch der letzten drei Lösungsmittel. Das entsprechende Polymer wird in das gleiche Lösungsmittel, das auch zur Lösung des Wirkstoffs verwendet wurde, gegeben. Die beiden Ansätze werden vereinigt und können in Gegenwart eines wasserentziehenden Kupplungsreagenz, gegebenenfalls unter Lichtausschluß, bei einem pH Wert im Bereich von 7 bis 9, bevorzugt 8 bis 8,5, und bei einer Temperatur von 0 bis 100° C, bevorzugt 20 bis 30° C, über einen Zeitraum von ca. 1 bis 29 Stunden inkubiert werden, vorzugsweise unter Rühren.

Als wasserentziehendes Kupplungsreagenz können Carbodiimide, Alkylphosphonsäureanhydride, Carbonyldiamine etc., zur Anwendung kommen. Besonders bevorzugt werden Carbonyldiimidazol und Dicyclohexylcarbodiimid verwendet.

Das entstandene Rohprodukt kann zur Aufreinigung mit einem Lösungsmittel, in dem das Polymer nicht löslich ist, ausgefällt werden. Hierzu können beispielsweise Tetrahydrofuran, Aceton, Dioxan und Alkohole verwendet werden. Eine weitere Reinigung kann mit Methoden zur Molekulargewichtsverteilung, wie z. B Ultrafiltration, Dialyse und Gelpermeation geschehen.

Nach den erfindungsgemäßen Verfahren erhält man ein polymerfixiertes Methotrexatprodukt mit einer Beladung von Methotrexat bzw. deren Analoga von 1 bis 85 %, bevorzugt 15 bis 75 %, bezogen auf das Gewicht des polymerfixierten Produkts. Ferner besitzt das Produkt einen Extinktionskoeffizienten zwischen 0,0001 l/mg und 0,05 l/mg, vorzugweise zwischen 0,005 l/mg und 0,03 l/mg, in wäßriger Lösung bei pH 7 bis 8,5 und einer Wellenlänge von 302 nm für Methotrexat und Methopterin, bzw. einen Extinktionskoeffizienten von 0,0005 l/mg bis 0,04 l/mg bei genanntem pH-Wert und einer Wellenlänge von 282 nm für Aminopterin.

Aus den erfindungsgemäßen polymerfixierten Methotrexaten kann der Wirkstoff durch einfache Hydrolyse langsam in den Organismus abgegeben werden im Gegensatz zur festeren Fixierung über funktionelle Amidgruppen, von denen der Wirkstoff nur durch enzymatische Spaltung freigesetzt werden kann.

In vivo werden mit diesen erfindungsgemäßen Polymer-Methotrexat-Konjugaten, z. B. in Mäusen mit L1210-Leukämie Lebensverlängerungen von > 125 %, bevorzugt > 150 %, gegenüber unbehandelten Kontrollen schon bei einmaliger Applikation (intraperitoneal i.p.) von 60 mg/kg Körpergewicht Methotrexat bzw. Derivaten, die erfindungsgemäß an das wasserlösliche, biokompatible hydroxylgruppentragende Polymer gebunden sind (Methotrexat- bzw. Derivat-Äquivalent), erreicht. Diese Werte wurden, wie in Beispiel 7 beschrieben, ermittelt. Unter den genannten Bedingungen sind Methotrexat oder die Derivate selbst nicht wirksam. Bei 330 mg/kg Methotrexat-bzw. Derivat-Äquivalent konnte bei 2 von 5 Tieren komplette Remission ohne Rückfall bis zum Versuchsabbruch nach 60 Tagen beobachtet werden. Diese Dosis liegt bereits über der $LD_{50}$ von freiem Methotrexat.

In vitro zeigen die erfindungsgemäßen Polymer-Methotrexat Konjugate außerdem höhere $IC_{50}$ als freies Methotrexat (z. B. gegen L1210, HT 29, A549-Zellen). In Verbindung mit den oben beschriebenen in vivo-Ergebnissen ist dies als ein Beweis für eine gewünschte langsame Freisetzung von Methotrexat anzusehen.

Im folgenden wird die Erfindung anhand von Beispielen erläutert. Die Prozentangaben beziehen sich, wenn nichts anderes angegeben, auf das Gewicht.

Beispiel 1: Darstellung eines Poly-methotrexat-poly-α,β-(2-hydroxyethyl)-D,L-aspartamid-Esters mit Hilfe von Carbonyldiimidazol

Methotrexat für alle Beispiele wurde von der Firma Sigma bezogen. Das Polymer Poly-α,β-(2-hydroxyethyl)-D,L-aspartamid wird nach P. Neri, G. Antoni, F. Benvenuti, F.Cocola, G. Gazzei, J. Med. Chem. 16, 893 (1973), dargestellt.

3 g Methotrexat werden in 15 ml eines Gemisches von Formamid : N,N-Dimethylformamid : $CH_2Cl_2$ (10 : 9 : 1) gelöst. Dazu gibt man eine Lösung von 300 mg Pyrrolidinopyridin und 2,44 g Carbonyldiimidazol in 5 ml des obigen Lösungsmittelgemisches. Die Reaktionsmixtur wird 1,5 h bei Raumtemperatur (RT) gerührt. Gleichzeitig werden 3,1 g Poly-α,β-(2-hydroxyethyl)-D,L-aspartamid (PHEA) in 9 ml des beschriebenen Lösungsmittelgemisches gelöst und bei RT gerührt. Die beiden Lösungen werden vereinigt und unter Lichtausschluß 20 h bei RT gerührt. Durch Eingießen in 250 ml Aceton wird das Rohprodukt ausgefällt. Der gelbe Niederschlag wird abgetrennt, mit Aceton gewaschen, getrocknet und dann in 25 ml wäßriger $NaHCO_3$-Lösung (pH 8 -9) aufgenommen.

Die bernsteingelbe wäßrige Lösung wird über eine Sephadex-Gelchromatographiesäule (PD10, Pharmacia) gegeben und durch Elution mit Wasser in eine niedermolekulare und eine hochmolekulare Fraktion aufgetrennt. Die hochmolekulare Fraktion wird gefriergetrocknet. Der Methotrexatgehalt wird durch UV-Spektroskopie bei $\lambda$ = 302 nm in wäßriger Lösung bestimmt. Das Produkt wird durch $^1H$-NMR, CHN-Analyse und Dünnschichtchromatographie charakterisiert. Das $^1H$-NMR in $D_2O$ entspricht einer Summe der Spektren von Methotrexat und PHEA, wobei aus den Integralverhältnissen auf den Belegungsgrad geschlossen werden kann. Der mit HPLC gemessene Belegungsgrad von 22 Gew.-% Methotrexat entspricht dem Ergebnis der UV-Bestimmung. Im DC in Diethylether laufen die Kopplungsreagenzien, während Methotrexat und Produkt bei Rf = 0 bleiben. Die Anwesenheit von freiem Methotrexat konnte durch Ultrafiltration mit Membranen verschiedener Ausschlußgrenzen ausgeschlossen werden.

Ausbeute: 3 g polymerfixiertes Methotrexat (ca. 20 % d. Th., bezogen auf das Methotrexat). Der größte Teil des nicht umgesetzten Methotrexats wird durch Umfällung aus der niedermolekularen Fraktion zurückgewonnen.

Beispiel 2: Darstellung eines Poly-methotrexat-dextran (40.000)-Esters mit Hilfe von Carbonyldiimidazol

Dextran 40.000 wurde von der Fa. Fluka, Buchs, Schweiz bezogen. 3 g Methotrexat werden in 10 ml eines Gemisches aus Formamid : N,N-Dimethylformamid : $CH_2Cl_2$ (10 : 9 : 1) gelöst und 2,39 g Carbonyldiimidazol sowie 0,29 g Pyrrolidinopyridin in 5 ml des obigen Lösungsmittelgemisches werden gemischt und 2 h bei RT gerührt. Dann gibt man dazu eine Lösung von 3,2 g Dextran 40.000 in 20 ml desselben Lösungsmittelgemisches und rührt 20 h bei RT unter Lichtausschluß. Das Rohprodukt wird in 350 ml trockenem Aceton ausgefällt, mit Aceton gewaschen und getrocknet.

Der Feststoff wird in 25 ml $H_2O$ aufgenommen und über eine PD10 -Gelchromatographiesäule gegeben. Elution mit $H_2O$ ergibt eine hoch- und eine niedermolekulare Fraktion. Die hochmolekulare Fraktion wird gefriergetrocknet und, wie unter Beispiel 1 beschrieben, analysiert. Rückgewinnung des nicht umgesetzten Methotrexats ist durch Umfällung möglich.

Ausbeute: 3 g polymerfixiertes Methotrexat
Belegung: 17,3 Gew.-% Methotrexat

**Beispiel 3:** Darstellung eines Poly-methotrexat-dextran (40.000)-Esters mit Hilfe von Dicyclohexylcarbodiimid (DCC)

Dextran 40.000 wurde von der Fa. Fluka, Buchs, Schweiz bezogen. 3 g Methotrexat werden in 10 ml eines Gemisches aus Formamid : N,N-Dimethylformamid : $CH_2Cl_2$ (10 : 9 : 1) gelöst und eine Lösung von 2,9 g DCC mit 1,8 g N,N-Dimethylaminopyridin (DMAP) in 5 ml Lösungsmittelgemisch (wie oben beschrieben) dazugegeben. Nach kurzem Rühren wird eine Lösung von 3,2 g Dextran 40.000 in 20 ml desselben Lösungsmittelgemisches zugegeben und 20 h bei RT unter Lichtausschluß gerührt. Nach Abfiltrieren eines Niederschlages wird das Rohprodukt durch Eingießen in 350 ml Aceton gefällt und mit Aceton gewaschen, dann getrocknet.

Der Feststoff wird in 30 ml $H_2O$ aufgenommen und über eine PD10-Gelpermeationschromatographiesäule gegeben und mit $H_2O$ eluiert. Die hochmolekulare Fraktion wird gefriergetrocknet und wie unter Beispiel 1 beschrieben analysiert.
Ausbeute: 3 g polymerfixiertes Methotrexat
Belegung: 8,5 Gew.-% Methotrexat;
Methotrexat-Rückgewinnung durch Umfällung.

**Beispiel 4:** Darstellung eines Poly-methotrexat-(α,β-(2-hydroxyethyl)-D,L-aspartamid/Polysuccinimid)-Esters

10 g (103 mmol) Polyanhydroasparaginsäure (Polysuccinimid) werden mit 1,83 g (30 mmol) 2-Aminoethanol nur partiell zu Poly-α,β-(2-hydroxyethyl)-D,L-aspartamid umgesetzt. Das NMR-spektroskopisch charakterisierte Polyanhydroasparaginsäure-co-α,β-(2-hydroxyethyl)-D,L-aspartamid enthält etwa 30 % Hydroxyethylgruppen und ist im Gegensatz zum Homo-Poly-α,β-(2-hydroxyethyl)-D,L-aspartamid, das in kaltem Wasser gelöst werden kann, nur noch in warmen Wasser löslich.

Die Reaktion wird analog Beispiel 1 durchgeführt, nur daß das Copolymer anstelle von Poly-α,β-(2-hydroxyethyl)-D,L-aspartamid eingesetzt wurde. Die eingesetzte Polymermenge hängt vom Poly-α,β-(2-hydroxyethyl)-D,L-aspartamid-Anteil des Copolymers ab. Auf 1 Mol Methotrexat wird jeweils Polymer entsprechend 1 Mol OH-Gruppen angesetzt. Das gelchromatographierte hochmolekulare Endprodukt enthält 20 Gew.-% gebundenes Methotrexat (über HPLC bestimmt). Ausbeute ca. 20 % d. Th., bezogen auf das eingesetzte Methotrexat.

**Beispiel 5:** Darstellung eines Poly-methotrexat-inulin-Esters

Die Reaktion wird analog Beispiel 1 durchgeführt, nur daß Inulin (Fluka AG, Buchs, Schweiz) anstelle von Poly-α,β-(2-hydroxyethyl)-D,L-aspartamid eingesetzt wurde. Auf 1 Mol Methotrexat wird eine Inulinmenge entsprechend 1 Mol Monomereneinheiten angesetzt. Das gelchromatographierte hochmolekulare Endprodukt enthält 68 Gew.-% gebundenes Methotrexat. Ausbeute ca. 70 % d. Th., bezogen auf das eingesetzte Methotrexat.

**Beispiel 6:** Darstellung eines Poly-methotrexat-poly-[(2-hydroxyethyl-amido)-lysinfumaramid]-Esters mit Hilfe von Carbonyldiimidazol

Die Darstellung des Polymers erfolgt analog DOS 3707369 Beispiel 2, wobei anstelle von Lysinmethylester (2-Hydroxyethyl)amidolysin eingesetzt wird.

3 g Methotrexat werden in 15 ml des in Beispielen 1 bis 3 beschriebenen Lösungsmittelgemisches gelöst und mit einer Lösung von 2,39 g Carbonyldiimidazol und 0,29 g Pyrrolidinopyridin in 5 ml des Lösungsmittelgemisches versetzt. Nach 2 h Rühren bei RT wird mit einer Lösung von 3,3 g des Polymers in 20 ml des Lösungsmittelgemisches versetzt und bei RT unter Lichtausschluß 20 h gerührt. Dann wird durch Eingießen in 300 ml Aceton gefällt, der Rückstand mit Aceton gewaschen und getrocknet. Der Feststoff wird in 25 ml $H_2O$ gelöst und über eine PD-10-Gelchromatographiesäule werden hoch- und niedermolekulare Fraktionen getrennt. Die getrocknete hochmolekulare Fraktion wird, wie unter Beispiel 1 beschrieben, analysiert.
Ausbeute: 3,1 g polymerfixiertes Methotrexat
Belegung: 19,1 Gew.-% Methotrexat;
Methotrexat- Rückgewinnung durch Umfällung.

**Beispiel 7:** Darstellung eines Poly-methotrexat(wasserlösliche Stärke)-Esters mit Hilfe von Carbonyldiimidazol

Verschiedene kommerziell erhältliche wasserlösliche Stärken wurden eingesetzt. 3 g Methotrexat werden in 15 ml des in Beispielen 1 bis 3 beschriebenen Lösungsmittelgemisches gelöst und mit einer Lösung von 2,39 g Carbonyldiimidazol und 0,29 g Pyrrolidinopyridin in 5 ml desselben Gemisches versetzt. Nach 2 h Rühren bei RT werden 3,2 g wasserlösliche Stärkefraktion in 30 ml des Lösungsmittelgemisches zugesetzt und die Reaktionslösung 20 h bei RT und Lichtausschluß gerührt. Durch Eingießen in 300 ml Aceton wird das Rohprodukt gefällt und, nach Waschen mit Aceton, getrocknet. Der Rückstand wird in 30 ml $H_2O$ aufgenommen und ultrafiltriert. Die hochmolekulare Phase (Retentat, Membranausschlußgrenze = 5000) wird gefriergetrocknet und, wie unter Beispiel 1 beschrieben, analysiert.
Ausbeute: 3 g polymerfixiertes Methotrexat
Belegung: 8 Gew.-% Methotrexat;
Methotrexat-Rückgewinnung durch Umfällung.

**Beispiel 8:** In vitro Wirkung von Methotrexat-Dextran-Ester auf Tumor-Zellinien.

Proliferationstest (Methotrexat-Reduktion)

L1210, A 549 oder HT 29 in exponentieller Wachstumsphase werden in einer Zelldichte von 5 x $10^3$ Zellen/ml in Medium "Rosswell Park Memorial Institute" (RPMI) 1640 in einer Mikrotiterplatte mit 96 Vertiefungen 72 Stunden mit unterschiedlicher Konzentration der Testsubstanz bei 37° C, 5 % $CO_2$ und 95 % relativer Luftfeuchte inkubiert. Kontrollexperimente erhalten anstelle der Testsubstanz lediglich Wachstumsmedium. Für jede Testsubstanz sowie für die Kontrolle werden 4fach Bestimmungen angesetzt. Nach 65 Stunden Inkubation werden 5 µl einer Methotrexat-Lösung (2,5 mg/ml in phosphatgepufferter Kochsalz-Lösung) zugegeben. In Gegenwart lebender Zellen wird Methotrexat zu einem dunkelroten unlöslichen Formazanfarbstoff reduziert. Diese Reaktion ist nach 7 Stunden (L1210 Zellen) bzw. nach 24 Stunden (A 549, HT 29 Zellen) beendet und das überstehende Medium wird sorgfältig abgesaugt. Der unlösliche Farbstoff wird durch Zugabe von 100 µl DMSO aufgelöst und die Extinktion der so entstehenden Lösung anschließend für jede Vertiefung in einem Multiscan Photometer 340 CC der Fa. Flow bei einer Wellenlänge von 492 nm gemessen.

Aus dem Verhältnis der Extinktionen behandelter und unbehandelter Zellen ergibt sich eine Dosis-Wirkungskurve, aus der die Konzentration, die gerade 50 % der Zellen ($IC_{50}$) abgetötet, abgelesen werden kann. Für wiederholte Untersuchungen beträgt der Variationskoeffizient weniger als 15 %.

Tabelle 1

| Substanz | Zelle | $IC_{50}$(µg/ml) |
|---|---|---|
| Methotrexat (MTX) | L1210<br>HT 29<br>A 549 | 0,01<br>0,008<br>0,01 |
| MTX-Dextran-Ester | L1219<br>HT 29<br>A 549 | 0,13<br>0,41<br>0,58 |

**Beispiel 9:** In vivo Wirksamkeit auf L1210 Leukämie in Mäusen

Tumorgewinnung:

Aszites Flüssigkeit wird 7 Tage nach der Tumor-Implantation unter sterilen Bedingungen aus DBA2

Mäusen entnommen (weiblich, 18 bis 20 g). Die Aszites Flüssigkeit wird dreimal mit PBS (phosphate buffered saline) gewaschen, gezählt und anschließend in PBS verdünnt bis zu einer Endkonzentration von $10^6$ Zellen pro 0,2 ml.

$10^6$ Zellen in 0,2 ml PBS werden DBA2 Mäusen (weiblich, 18 bis 20 g) intraperitoneal appliziert. Dieser Transfer wird einmal wöchentlich wiederholt.

Ermittlung des antitumoralen Effektes:

$10^5$ Zellen der Aszites Flüssigkeit in 0,2 ml PBS werden BDF1 Mäusen (weiblich, 18 bis 20 g) intraperitoneal appliziert. 6 Tiere werden für jede Substanzkonzentration sowie für die Kontrolle eingesetzt.

a) Die Tiere werden an Tag 1 und Tag 5 nach der Tumorzellenimplantation gewogen. Gewichtsverlust von mehr als 20 % an Tag 5 wird als Indikator eines toxischen Substanzeffektes verwendet.

b) Am Ende des Experiments (Tod aller Tiere oder Erreichen von Tag 60) wird die mediane Überlebenszeit der Behandlungsgruppen bestimmt, sofern diese 65 % überlebende Tiere am Tag 5 enthielten. Die Ermittlung der medianen Überlebenszeit erfolgt entsprechend der Formel:

$$\text{median survival time (MST)} = \frac{(X + Y)}{2}$$

In dieser Formel bedeutet X den frühesten Tag, an dem die Zahl der überlebenden Tiere N/2 und Y den frühesten Tag, an dem die Zahl der überlebenden Tiere (N/2)-1 ist. In dem Fall, daß N eine ungerade Zahl ist, entspricht die mediane Überlebenszeit dem Zeitpunkt X.

Die mediane Überlebenszeit wird ausschließlich für im Verlauf des Experiments sterbende Tiere bestimmt. Geheilte Tiere (long time survivors, LTS) werden von der Bestimmung der medianen Überlebenszeit ausgeschlossen und getrennt aufgeführt.

Aus der medianen Überlebenszeit der behandelten Gruppen ($MST_{Tumor}$) und Kontrollgruppen ($MST_{Control}$) wird der antitumorale Effekt-Tumor/control (T/C) entsprechend der Formel

$$T/C\ \% \quad \frac{MST_T}{MST_C} \quad x\ 100$$

bestimmt. T/C Werte von mehr als 125 % werden als Indikator einer signifikanten antitumoralen Wirksamkeit der Testverbindung angesehen. Die Dosis, die jeweils den größten antitumoralen Effekt bewirkt (optimale Dosierung) ist in Tab. 2 aufgeführt. Noch lebende Tiere an Tag 60 werden als geheilt betrachtet (LTS).

Ergebnisse: s. Tabelle 2

Diskussion:

Aufgrund dieser Ergebnisse läßt sich ersehen, daß die veresterten Methotrexatpolymere bei einmaliger i.p. Applikation dem reinen Methotrexat überlegen sind, was auf eine "slow release" Wirkung der Polymere hindeutet.

Tabelle 2

| Substanz | Tumor | | optimale Dosis (mg polymerfixiertes Produkt/kg Körpergewicht) | Wirkung (T/C %) | Heilungen | toxisch |
|---|---|---|---|---|---|---|
| MTX-PHEA (19 Gew.% MTX) | L1210 | 1xip/ip<br>3xip/ip/Q3D | 221<br>95 | 192<br>326 | 1/6<br>4/6 | -<br>2 |
| MTX-Dex. (20 Gew.% MTX) | L1210 | 1xip/ip<br>3xip/ip/Q3D | 330<br>250 | 265<br>404 | 2/6<br>5/6 | 3<br>1 |
| MTX<br>(z.Vergl.) | L1210 | 1xip/ip<br>3xip/ip/Q3D | 100<br>80 | 123<br>378 | -<br>5/6 | -<br>1 |

ip = intraperitoneal
Q3 = Applikation im Abstand von 3 Tagen

## Ansprüche

1. Polymerfixiertes Methotrexat oder Methotrexatderivate gekennzeichnet durch
- eine Esterbindung der α- und/oder γ-Carboxylgruppe des Methotrexat oder der Methotrexatderivate mit Hydroxylgruppen von wasserlöslichen biokompatiblen Polymeren
- eine Belegung von 1 bis 85 % Methotrexat oder Methotrexatderivate
- Bewirkung einer Lebensverlängerung von mehr als 125 % gegenüber unbehandelten Kontrollen in Mäusen mit L1210-Leukämie bei einmaliger intraperitonealer Applikation von 60 mg Methotrexat oder Methotrexatderivat, das an obengenantes Polymer fixiert ist, pro kg Körpergewicht.

2. Verbindung nach Anspruch 1, dadurch gekennzeichnet, daß das Polymer vor der Beladung einen Anteil an ionisierbaren Gruppen unter 10 Mol-% aufweist.

3. Verbindung nach Anspruch 1 oder 2, dadurch gekennzeichnet, daß das Polymer
a) ein Poly-α,β-(2-hydroxyethyl)-D,L-aspartamid der Formel I ist,

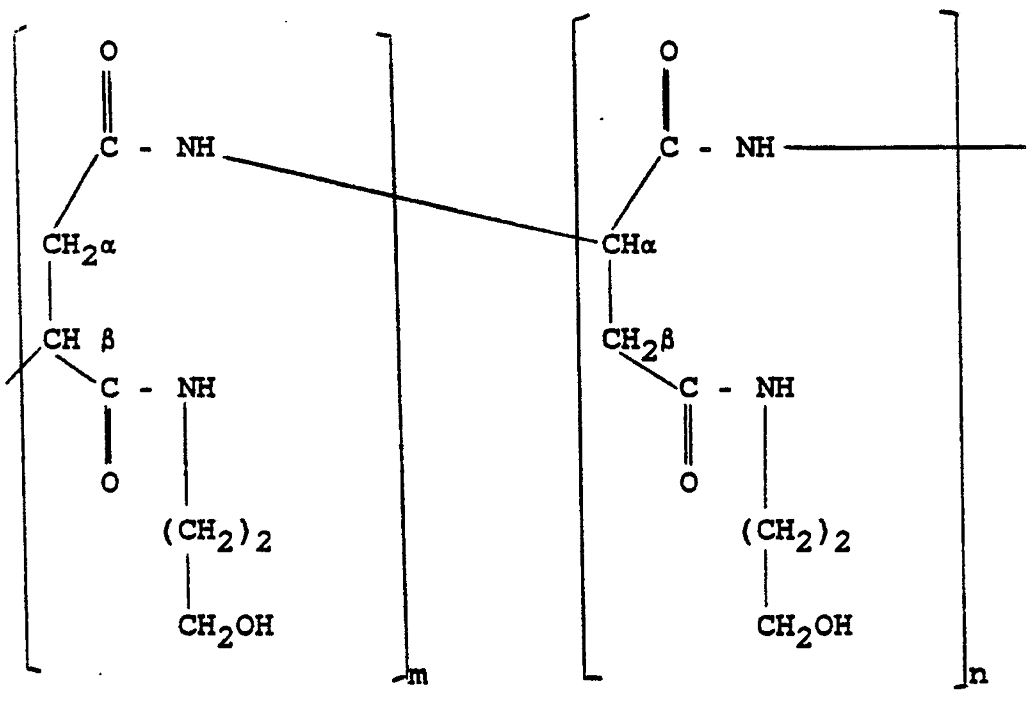

I

in der das Verhältnis von m zu n 0:1 bis 1:0 ist, mit einem Molekulargewicht von 2000 bis 100.000,

b) ein Polylysinfumaramid oder Glutaramid der Formel II,

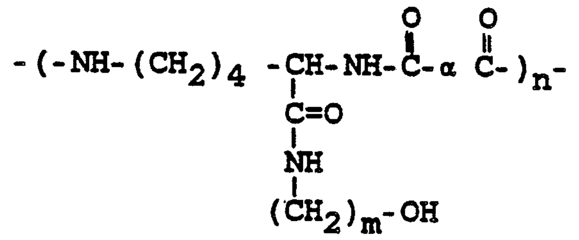

II

in der X - $(CH_2)_3$- oder -CH = CH- ,

m eine Zahl von 1 bis 10 und

n eine Zahl von 5 bis 2000 bedeutet,

mit einem Molekulargewicht zwischen 1000 und 300.000

c) ein Poly-α,β-(2-hydroxyethyl)-D,L-aspartamid/Polysuccinimid der Formel III,

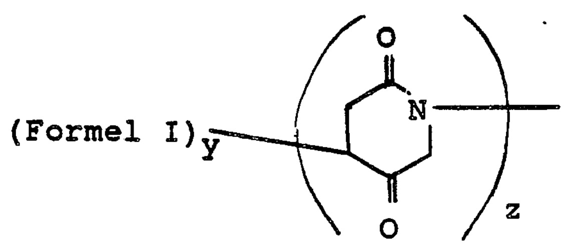

III

in der das Verhältnis von y zu z im Bereich von etwa 0,99:0,01 bis 0,01:0,99 liegt.

d) ein Dextran mit einem mittleren Molekulargewicht zwischen 1000 und 200.000

e) eine Stärkefraktion mit einem mittleren Molekulargewicht zwischen 1000 und 200.000 oder

f) Inulin

ist.

4. Ein Verfahren zur Herstellung des polymerfixierten Methotrexat oder der polymerfixierten Methotrexatderivate nach Anspruch 1, dadurch gekennzeichnet, daß das α- und/oder γ-Carboxylgruppentragende Methotrexat oder die entsprechenden Derivate des Methotrexats in Gegenwart wasserentziehender Kupplungsreagenzien mit einem hydroxylgruppentragenden, wasserlöslichen, biokompatiblen Polymeren zur Reaktion gebracht wird.

5. Verfahren nach Anspruch 4, dadurch gekennzeichnet, daß als Polymer

a) ein Poly-α,β-(2-hydroxyl)- D,L,-aspartamid der Formel I

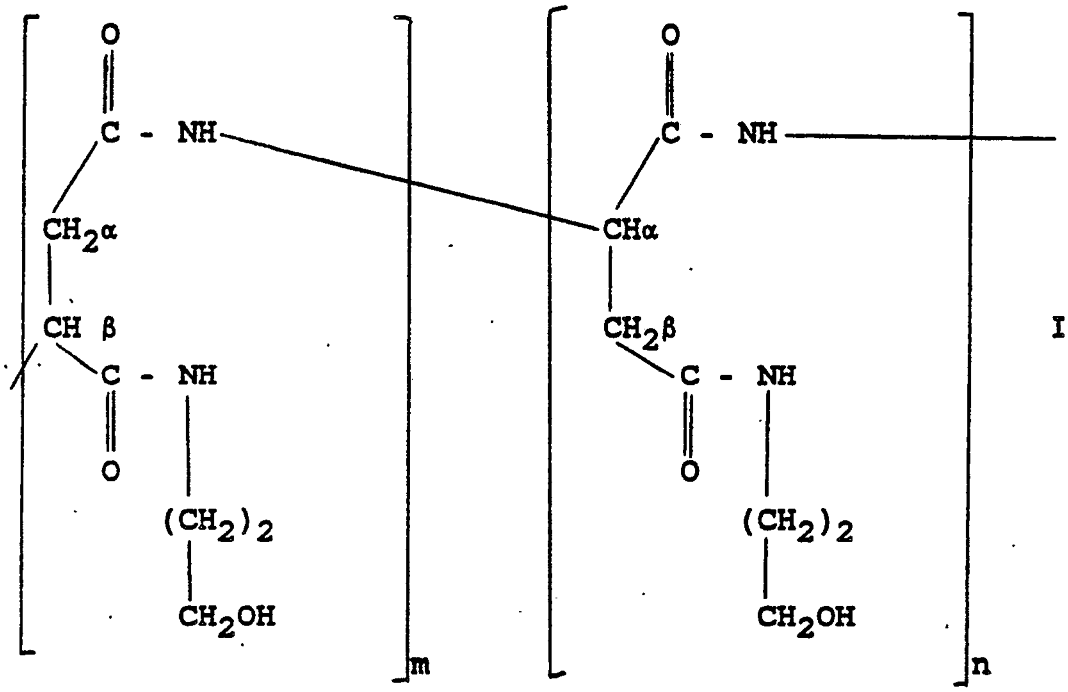

I

in der das Verhältnis von m zu n 0:1 bis 1:0 ist, mit einem Molekulargewicht von 2000 bis 100.000;

b) ein Polylysinfumaramid oder Glutaramid der Formel II,

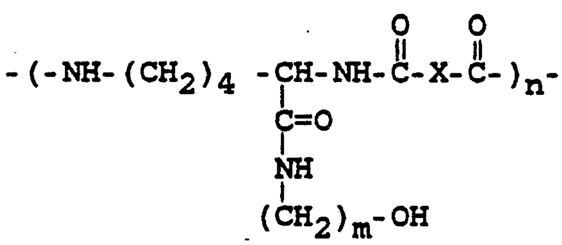

II

in der X - $(CH_2)_3$ oder -CH = CH-,
m eine Zahl von 1 bis 10 und
n eine Zahl von 5 bis 1000 bedeutet,
mit einem Molekulargewicht zwischen 1000 und 300.000

c) ein Poly-α,β-(2-hydroxyethyl)-D,L-aspartamid/Polysuccinimid der Formel III,

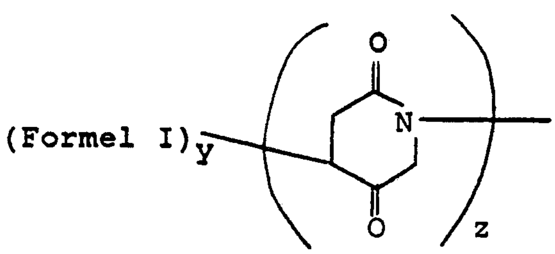

III

in der das Verhältnis von y zu z im Bereich von etwa 0,99:0,01 bis 0,01:0,99 liegt.

d) ein Dextran mit einem mittleren Molekulargewicht zwischen 1000 und 200.00 und

e) eine Stärkefraktion mit einem mittleren Molekulargewicht zwischen 1000 und 200.000

f) Inulin

eingesetzt wird.

6. Verfahren nach Anspruch 4 oder 5, dadurch gekennzeichnet, daß das als Kupplungsreagenz Carbonyldiimidazol oder Dicyclohexylcarbodiimid eingesetzt wird.

7. Verfahren nach einem oder mehreren der Ansprüche 4 bis 6, dadurch gekennzeichnet, daß die Reaktion bei einem pH-Wert von 7 bis 9 und einer Temperatur von 0 bis 100 °C stattfindet.

8. Verwendung des polymerfixierten Methotrexat oder der polymerfixierten Methotrexatderivate nach Anspruch 1 als Tumortherapeutikum.

Patentansprüche für folgende Vertragsstaaten: ES,GR

1. Verfahren zur Herstellung des polymerfixierten Methotrexat oder der polymerfixierten Methotrexatderivate mit

- einer Esterbindung der α- und/oder γ-Carboxylgruppe des Methotrexat oder der Methotrexatderivate mit Hydroxylgruppen von wasserlöslichen biokompatiblen Polymeren
- einer Belegung von 1 bis 85 % Methotrexat oder Methotrexatderivate,

dadurch gekennzeichnet, daß das α- und/oder γ-Carboxylgruppentragende Methotrexat oder die entsprechenden Derivate der Methotrexats in Gegenwart wasserentziehender Kupplungsreagenzien mit einem hydroxylgruppentragenden, wasserlöslichen, biokompatiblen Polymern zur Reaktion gebracht wird.

2. Verfahren nach Anspruch 1, dadurch gekennzeichnet, daß als Polymer

a) ein Poly-α,β-(2-hydroxyl)-D,L-aspartamid der Formel I ist,

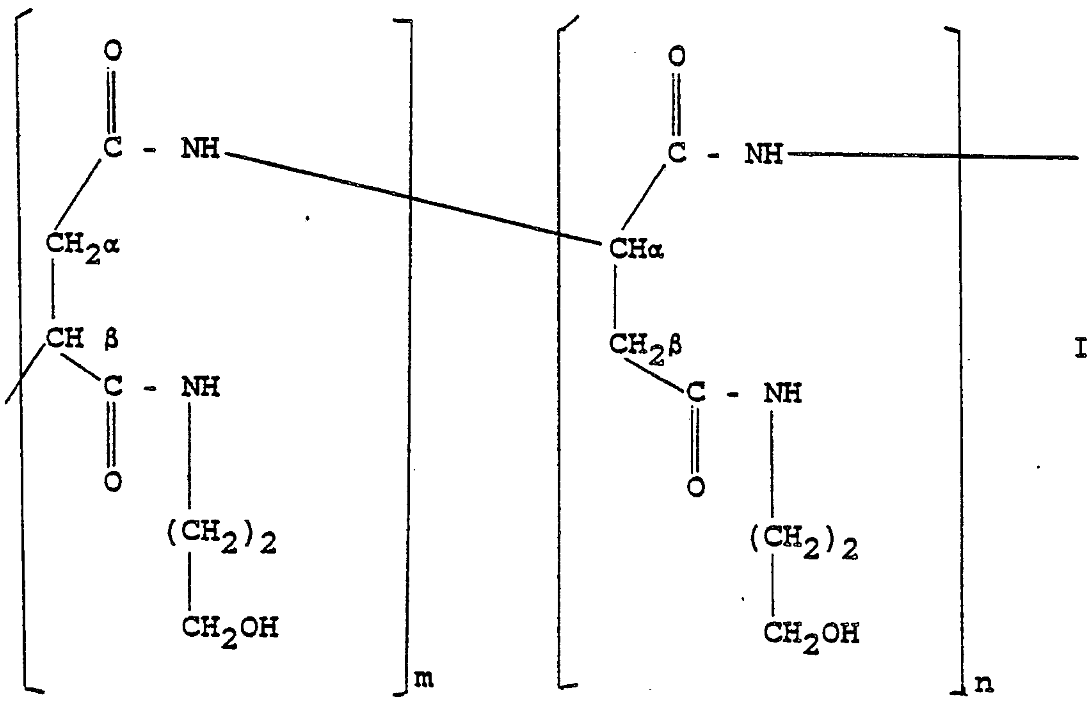

I

in der das Verhältnis von m zu n 0:1 bis 1:0 ist, mit einem Molekulargewicht von 2000 bis 100.000,

b) ein Polylysinfumaramid oder Glutaramid der Formel II,

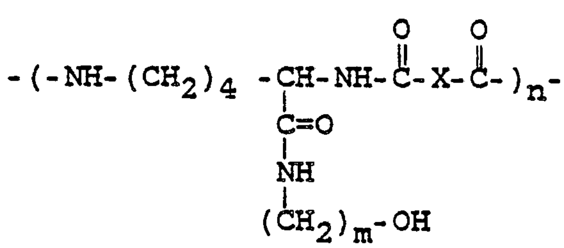

II

in der X $-(CH_2)_3-$ oder $-CH=CH-$,

m eine Zahl von 1 bis 10 und

n eine Zahl von 5 bis 2000 bedeutet,

mit einem Molekulargewicht zwischen 1000 und 300.000

c) ein Poly-α,β-(2-hydroxyethyl)-D,L-aspartamid/Polysuccinimid der Formel III,

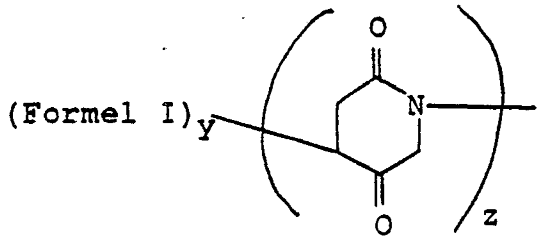

III

in der das Verhältnis von y zu z im Bereich von etwa 0,99:0,01 bis 0,01:0,99 liegt.

d) ein Dextran mit einem mittleren Molekulargewicht zwischen 1000 und 200.00 und

e) eine Stärkefraktion mit einem mittleren Molekulargewicht zwischen 1000 und 200.000

f) Inulin

eingesetzt wird.

3. Verfahren nach Anspruch 1 oder 2, dadurch gekennzeichnet, daß das als Kupplungsreagenz Carbonyldiimidazol oder Dicyclohexylcarbodiimid eingesetzt wird.

4. Verfahren nach einem oder mehreren der Ansprüche 1 bis 3, dadurch gekennzeichnet, daß die Reaktion bei einem pH-Wert von 7 bis 9 und einer Temperatur von 0 bis 100 °C stattfindet.

5. Verwendung des nach Anspruch 1 erhältlichen polymerfixierten Methotrexat oder der polymerfixierten Methotrexatderivate als Tumortherapeutikum.